# EUROPEAN PATENT SPECIFICATION

(11) **EP 4 563 100 B1**
(45) Date of publication and mention of the grant of the patent: **06.05.2026**
(21) Application number: 23870511.5
(22) Date of filing: 20.09.2023
(51) Int. Cl.: A61B 17/16, A61B 17/17

(54) **COOLABLE ABRASIVE DRILL FOR MINIMALLY INVASIVE SPINE SURGERY**
KÜHLBARER ABRASIVBOHRER FÜR DIE MINIMALINVASIVE WIRBELSÄULENCHIRURGIE
FORET ABRASIF REFROIDISSABLE POUR CHIRURGIE MINI-INVASIVE DE LA COLONNE VERTÉBRALE

(30) Priority: 30.09.2022 CN 202222621844 U
(43) Date of publication of application: 04.06.2025
(73) Proprietor: Bangshi Medical Technology Co., Ltd., Taizhou, Jiangsu 225316 (CN)
(72) Inventor: HE, Chengdong, Taizhou, Jiangsu 225316 (CN); YUE, Xin, Taizhou, Jiangsu 225316 (CN); DONG, Qingpeng, Taizhou, Jiangsu 225316 (CN)
(74) Representative: Bayramoglu et al.
(86) International application number: PCT/CN2023/119934
(87) International publication number: WO 2024/067279

(56) References cited:
- CN-A- 111 973 249
- CN-U- 212 438 753
- CN-U- 214 017 702
- CN-U- 215 018 430
- CN-U- 218 305 024
- CN-U- 219 289 589
- CN-U- 219 962 985
- US-A1- 2015 201 918
- US-A1- 2017 319 217

## Description

### TECHNICAL FIELD

The utility model relates to the technical field of medical appliances, and in particular to a grinding drill with a cooling function for a minimally invasive spinal surgery.

### BACKGROUND TECHNOLOGY

The description of the background in the present application applies to related art of the present application, and is merely intended to illustrate the content of and facilitate understanding of the present application. It should not be deemed that the applicant clearly regards or is presumed to regard the description as belonging to the prior art of the present application on the filing date of the first application.

During spine grinding operations in surgical procedures, traditional manual grinders have problems such as low efficiency, inconvenience in observing the surgical site, and difficulty in controlling the force. As a result, it is difficult to remove a spine with low toughness around the surgical site, and it is easy to cause damage to a soft tissue with high toughness around, especially that adjacent to the spine, thereby affecting the recovery of the surgical site.

Traditional scrappers and grinding drills cannot meet the requirements of fine surgeries such as spine drilling due to their sizes. In addition, during high-speed rotation, external physiological saline needs to be introduced to cope with the heating problem of the grinding rod. The multi-instrument application has strict requirements for the surgical space due to the complexity of the spine area. However, the existing power system scrappers and grinding drills increase the surgical risk. For example, Chinese patent application CN211243576U discloses a grinding drill for a neurosurgery surgery. Mainly, a hollow tube is provided outside a grinding rod to introduce physiological saline for cooling. When in use, the external physiological saline needs to be continuously introduced. However, due to the limited surgical space of the spine, failure to timely extract the discharged physiological saline will seriously affect the surgical field of vision. Therefore, the existing power system scrappers and grinding drills are not suitable for drilling the spine. CN215018430U discloses an eccentric sheath grinding head with spiral cooling water channel and coaxial multi-tube structure.

At present, instrument cooling is still an urgent problem to be solved for spinal surgeries, especially those involving tissue scrapping and grinding.

### CONTENT OF THE UTILITY MODEL

An objective of the utility model is to provide a grinding drill with a cooling function for a minimally invasive spinal surgery. The utility model solves the problems that the existing grinders for minimally invasive surgeries require a large amount of coolant and cannot effectively cool the grinding rod.

To solve the technical problems, the utility model adopts the following technical solution.

A grinding drill with a cooling function for a minimally invasive spinal surgery includes a grinder fixture, a grinding rod assembly, and a grinder, where the grinder fixture includes a housing assembly and a sleeve that is detachably connected inside the housing assembly; one end of the grinding rod assembly extends into the housing assembly and is connected to the sleeve through a connecting shaft; an inner grinding rod is rotatably provided in the grinding rod assembly; the grinder runs through the other end of the grinding rod assembly and is connected to the inner grinding rod; an end of the inner grinding rod away from the grinder extends into the sleeve and is rotatably connected to the sleeve through a transmission element; and the transmission element is connected to an external power handle through an interface of the sleeve; and
a spiral cooling passage communicated with a water injection hole of the sleeve is provided between an inner wall of the housing assembly and an outer wall of the sleeve; the grinding rod assembly is provided with a water inlet; and the water inlet is communicated with the cooling passage through a chamber inside the housing assembly.

In the utility model, the transmission element is connected to the external power handle through the interface, and the external power handle drives the inner grinding rod and the grinder to rotate and drill the spine. The external power handle is provided with an inlet pipe. When the external power handle is inserted into the interface and connected to the transmission element, the inlet pipe of the external power handle is communicated with the water injection hole of the sleeve, and the cooling water is introduced into the cooling passage and the chamber through the water injection hole. The cooling water enters the grinding rod assembly through the water inlet and cools the grinding rod assembly and the inner grinding rod. The cooling water finally flows through the grinder from the grinding rod assembly to cool the grinder, thereby reducing thermal damage to the tissue during the grinding process.

The grinding rod assembly includes a fourth outer tube, a third outer tube, a second outer tube and a first outer tube that are arranged sequentially from inside to outside; and the fourth outer tube, the third outer tube, the second outer tube and the first outer tube are sequentially communicated from outside to inside to form a cooling water passage for cooling the grinder and the inner grinding rod.

A cavity is formed between each two adjacent outer tubes; the water inlet is located on an outer wall of an end of the first outer tube adjacent to the connecting shaft; a side wall of the second outer tube is provided with a water guide passage that is communicated with the cavity between the first outer tube and the second outer tube; the third outer tube is provided with a water guide hole that is communicated with the cavity between the second outer tube and the third outer tube; and an end of each of the fourth outer tube and the third outer tube adjacent to the grinder is provided with a water outlet that is communicated with the cavity.

In the utility model, the cooling water enters the cavity between the first outer tube and the second outer tube through the water inlet of the first outer tube, and enters the cavity between the second outer tube and the third outer tube through the water guide passage on the outer wall of the second outer tube. The cooling water enters the cavity between the third outer tube and the fourth outer tube through the water guide hole of the third outer tube, and finally flows towards the grinder through the water outlet passage to cool the grinder.

Further, preferably, the housing assembly includes a fastening element and a water-cooled housing that is detachably connected to the fastening element; the fourth outer tube, the third outer tube, the second outer tube, and the first outer tube all extend into the fastening element and are connected to the connecting shaft through the first outer tube; the chamber is a gap between the fastening element and the connecting shaft; and the cooling passage is located between an inner wall of the water-cooled housing and the outer wall of the sleeve.

In the utility model, the fastening element is configured to strengthen the connection between the grinding rod assembly and the water-cooled housing, thereby improving the connection stability between the grinding rod assembly and the grinder fixture.

Further, preferably, an end of the water-cooled housing away from the fastening element is clamped to a locking element configured to lock the external power handle.

In the utility model, when the interface of the sleeve is connected to the external power handle, the locking element locks the sleeve, the water-cooled housing, and the external power handle, thereby improving the connection stability.

Further, preferably, an outer wall of the inner grinding rod is wrapped by an anti-friction tube; and the fourth outer tube is located outside the anti-friction tube.

In the utility model, the anti-friction tube reduces the friction between the inner grinding rod and the fourth outer tube.

Further, preferably, a gap is formed between the fourth outer tube and the grinder.

The gap is designed to prevent an end position of the fourth outer tube adjacent to the grinder from being tangled by a tissue.

Further, preferably, the grinder is connected to the inner grinding rod through a steel tube; and an end of the grinder away from the inner grinding rod forms an emery spherical grinding head, conical grinding head, or grinding head with a cutting edge.

In the utility model, different grinders can be selected according to different surgical environments.

Further, preferably, the transmission element includes a bearing provided inside the sleeve and a coupling in a rotational fit with the bearing; the coupling is connected to the inner grinding rod; and the coupling is connected to the external power handle through the interface.

In the utility model, the inner grinding rod is connected to the coupling, and the coupling is connected to the external power handle through the interface. The external power handle is activated to drive the coupling to rotate around the bearing, thereby driving the inner grinding rod and the grinder to rotate for grinding operations.

Further, preferably, a sealing ring is provided between the connecting shaft and the sleeve.

In the utility model, the sealing ring is configured to connect the connecting shaft to the sleeve in a sealed manner, thereby preventing the cooling water from entering the connecting shaft to cause the impact on the rotation of the inner grinding rod.

Compared with the prior art, the utility model has the following beneficial effects:
1. In the utility model, the transmission element is connected to the external power handle through the interface, and the external power handle drives the inner grinding rod and the grinder to rotate and drill the spine. The inlet pipe of the external power handle is communicated with the water injection hole, and the cooling water is introduced into the cooling passage and the chamber through the water injection hole. The cooling water enters the grinding rod assembly through the water inlet and cools the grinding rod assembly and the inner grinding rod. The cooling water finally flows through the grinder from the grinding rod assembly to cool the grinder, thereby reducing thermal damage to the tissue during the grinding process.
2. In the utility model, the coaxial multi-tube structure of the grinding rod assembly reduces the amount of the cooling water and undertakes the delivery of the cooling water in the grinder fixture, replacing a traditional external cooling pipe. The utility model realizes cooling of the high-speed grinding drill, improves the utilization of the space in surgeries of complex cavities, and accelerates the surgical process.

### DESCRIPTION OF THE DRAWINGS

FIG. 1 is a structural diagram of a grinding drill with a cooling function according to the utility model;
FIG. 2 is a section view of the grinding drill with the cooling function according to the utility model;
FIG. 3 is a specific structural diagram of a grinder fixture; and
FIG. 4 is a structural diagram of a grinding rod assembly, an inner grinding rod, and a grinder.

Reference Numerals: 1. grinder fixture; 11. housing assembly; 111. fastening element; 112. water-cooled housing; 113. locking element; 12. sleeve; 13. connecting shaft; 131. sealing ring; 14. interface; 15. water inlet; 16. cooling passage; 17. chamber; 2. grinding rod assembly; 21. water inlet; 22. fourth outer tube; 23. third outer tube; 231. water guide hole; 24. second outer tube; 241. water guide passage; 25. first outer tube; 26. water outlet; 3. grinder; 31. steel tube; 4. inner grinding rod; 5. transmission element; 51. bearing; 52. coupling; and 6. anti-friction tube.

### SPECIFIC IMPLEMENTATIONS

The technical solutions of the utility model are clearly and completely described with reference to the drawings. Apparently, the described embodiments are merely some rather than all of the embodiments of the utility model. All other embodiments obtained by those having ordinary skill in the art based on the embodiments of the utility model without creative efforts shall fall within the protection scope of the utility model.

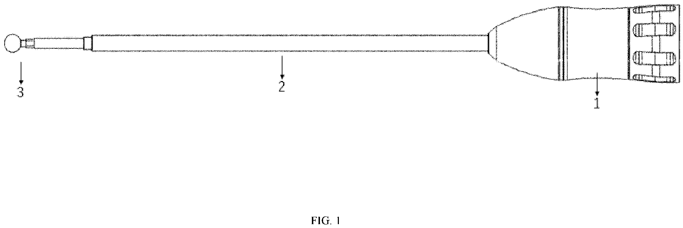

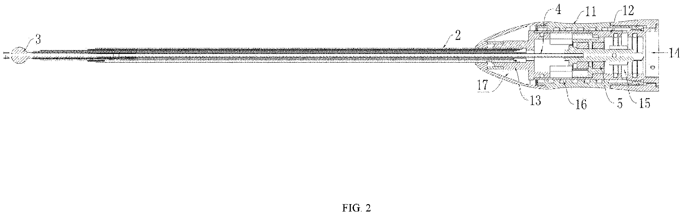

As shown in FIGS. 1 to 4, a grinding drill with a cooling function for a minimally invasive spinal surgery includes grinder fixture 1, grinding rod assembly 2, and grinder 3. The grinder fixture 1 includes housing assembly 11 and sleeve 12 that is detachably connected inside the housing assembly 11. An outer wall of the sleeve 12 is provided with an external thread. A corresponding position of water-cooled housing 112 in the housing assembly 11 is provided with an internal thread that matches the external thread to achieve a detachable connection. One end of the grinding rod assembly 2 extends into the housing assembly 11 and is connected to the sleeve 12 through connecting shaft 13. Sealing ring 131 is provided between the connecting shaft 13 and the sleeve 12. Specifically, an end of the connecting shaft 13 away from the grinding rod assembly 2 is embedded into the sleeve 12. An outer wall of the connecting shaft 13 is provided with a circular groove, and the circular groove is configured to accommodate the sealing ring 131, such that the sleeve 12 and the connecting shaft 13 are in a sealed state to prevent cooling water from entering into the sleeve 12 to interfere with transmission element 5.

Inner grinding rod 4 is rotatably provided in the grinding rod assembly 2. The grinder 3 runs through the other end of the grinding rod assembly 2 and is connected to the inner grinding rod 4. An end of the inner grinding rod 4 away from the grinder 3 extends into the sleeve 12 and is rotatably connected to the sleeve 12 through the transmission element 5. The transmission element 5 is connected to an external power handle through interface 14 of the sleeve 12. The transmission element 5 includes bearing 51 provided inside the sleeve 12 and coupling 52 in a rotational fit with the bearing 51. The coupling 52 is connected to the inner grinding rod 4, and the coupling 52 is connected to the external power handle through the interface 14. The external power handle is a driving component of an external host system. The external power handle drives the coupling 52 to rotate around the bearing 51, which in turn drives the inner grinding rod 4 and the grinder 3 to rotate and drill a spine.

Spiral cooling passage 16 communicated with water injection hole 15 of the sleeve 12 is provided between an inner wall of the housing assembly 11 and the outer wall of the sleeve 12. The external power handle is inserted into the interface 14 to connect the coupling 52. A water inlet pipe of the external power handle is inserted into the water injection hole 15 so as to introduce the cooling water into the water injection hole 15. The grinding rod assembly 2 is provided with water inlet 21. The water inlet 21 is communicated with the cooling passage 16 through chamber 17 inside the housing assembly 11. The cooling water enters the cooling passage 16 from the water injection hole 15, flows through the chamber 17, and flows into the grinding rod assembly 2 from the water inlet 21 to cool the grinding rod assembly 2, the inner grinding rod 4, and the grinder 3, thereby reducing thermal damage to the tissue during the grinding process.

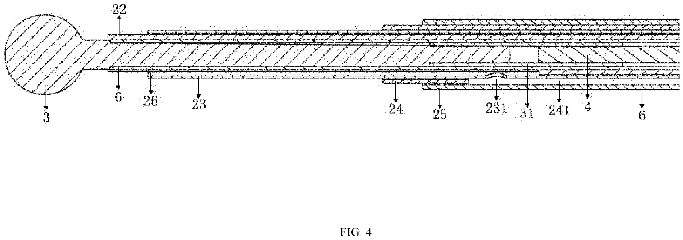

As shown in FIG. 4, the grinding rod assembly 2 includes fourth outer tube 22, third outer tube 23, second outer tube 24 and first outer tube 25 that are arranged sequentially from inside to outside. The fourth outer tube 22, the third outer tube 23, the second outer tube 24 and the first outer tube 25 are sequentially communicated from outside to inside to form a cooling water passage for cooling the grinder 3 and the inner grinding rod 4. A cavity is formed between each two adjacent outer tubes. The water inlet 21 is located on an outer wall of an end of the first outer tube 25 adjacent to the connecting shaft 13. A side wall of the second outer tube 24 is provided with water guide passage 241 that is communicated with the cavity between the first outer tube 25 and the second outer tube 24. The third outer tube 23 is provided with water guide hole 231 that is communicated with the cavity between the second outer tube 24 and the third outer tube 23. An end of each of the fourth outer tube 22 and the third outer tube 23 adjacent to the grinder 3 is provided with water outlet 26 that is communicated with the cavity.

The cooling water enters the cavity between the first outer tube 25 and the second outer tube 24 through the water inlet 21. The cooling water inside the cavity between the first outer tube 25 and the second outer tube 24 enters the cavity between the second outer tube 24 and the third outer tube 23 through the water guide passage 241. The cooling water in the cavity between the second outer tube 24 and the third outer tube 23 is guided into the cavity between the fourth outer tube 22 and the third outer tube 23 through the water guide hole 231, and finally reaches the grinder 3 from the water outlet 26. In this embodiment, the cooling water is medical sterile physiological saline. The cooling water passage for cooling the grinder 3 and the inner grinding rod 4 is formed by the cavity between the first outer tube 25 and the second outer tube, the water guide passage 241, the cavity between the second outer tube 24 and the third outer tube 23, the water guide hole 231, the cavity between the third outer tube 23 and the fourth outer tube 22, and the water outlet 26.

Preferably, a gap is formed between the fourth outer tube 22 and the grinder 3, and the gap is provided to prevent an end position of the fourth outer tube 22 adjacent to the grinder 3 from being tangled by a tissue.

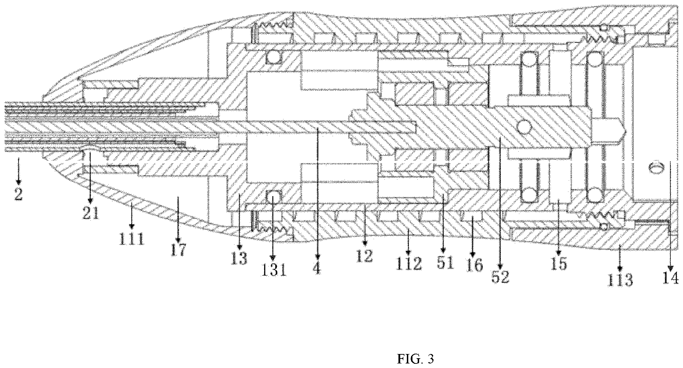

As shown in FIG. 3, the housing assembly 11 includes fastening element 111 and the water-cooled housing 112 that is detachably connected to the fastening element 111. The fastening element 111 is configured to strengthen the connection stability between the grinding rod assembly 2 and the grinder fixture 1. An inner wall of an end of the fastening element 111 adjacent to the water-cooled housing 112 is provided with an internal thread, and a corresponding position of the water-cooled housing 112 is provided with an external thread that matches the internal thread, thereby achieving a detachable connection. The fourth outer tube 22, the third outer tube 23, the second outer tube 24, and the first outer tube 25 all extend into the fastening element 111 and are connected to the connecting shaft 13 through the first outer tube 25. The chamber 17 is a gap between the fastening element 111 and the connecting shaft 13. The cooling passage 16 is located between an inner wall of the water-cooled housing 112 and the outer wall of the sleeve 12.

An end of the water-cooled housing 112 away from the fastening element 111 is clamped to locking element 113 configured to lock the external power handle. An outer wall of the end of the water-cooled housing 112 away from the fastening element 111 is provided with a clamping groove. An inner wall of the locking element 113 is correspondingly provided with a circular protrusion that matches the clamping groove, thereby achieving a clamping purpose. The locking element 113 clamps the sleeve 12, the water-cooled housing 112, and the external power handle to improve the connection stability. The locking element 113 is provided with a plurality of through holes for an inlet pipe of the external power handle to pass through.

In order to reduce the friction between the fourth outer tube 22 and the inner grinding rod 4, an outer wall of the inner grinding rod 4 is wrapped by anti-friction tube 6. The fourth outer tube 22 is located outside the anti-friction tube 6. The anti-friction tube 6 is made of an existing polytetrafluoroethylene tube, and the polytetrafluoroethylene tube has the properties such as high temperature resistance, friction resistance, and corrosion resistance.

As shown in FIG. 4, the grinder 3 is connected to the inner grinding rod 4 through steel tube 31. An end of the grinder 3 away from the inner grinding rod 4 forms an emery spherical grinding head, conical grinding head, or grinding head with a cutting edge. Different grinders can be selected according to different surgical environments.

In the utility model, when in use, the external power handle is inserted into the interface 14 and connected to the coupling 52. The external power handle drives the inner grinding rod 4 and the grinder 3 to rotate and drill the spine. The inlet pipe of the external power handle passes through the through holes of the locking element 113 and is communicated with the water injection hole 15. The cooling water introduced by the water injection hole 15 further enters the grinding rod assembly 2 through the cooling passage 16, the chamber 17, and the water inlet 21. The coaxial multi-tube structure of the grinding rod assembly 2 increases the flow time of the cooling water, allowing for full contact between the cooling water and the grinding rod assembly 2. Compared with a traditional external cooling pipe, in the utility model, the amount of the cooling water used by the grinding rod assembly 2 within the same time is reduced, and the cooling water can fully contact the grinding rod assembly 2, thereby effectively cooling the grinding rod assembly 2 and the inner grinding rod 4 and further cooling the grinder 3, so as to achieve cooling of the high-speed grinding drill. The utility model improves the utilization of the space in complex surgeries and accelerates the surgical process.

## Claims

1. A grinding drill with a cooling function for a minimally invasive spinal surgery, comprising a grinder fixture (1), a grinding rod assembly (2), and a grinder (3), wherein the grinder fixture (1) comprises a housing assembly (11) and a sleeve (12) that is detachably connected inside the housing assembly (11); one end of the grinding rod assembly (2) extends into the housing assembly (11) and is connected to the sleeve (12) through a connecting shaft (13); an inner grinding rod (4) is rotatably provided in the grinding rod assembly (2); the grinder (3) runs through the other end of the grinding rod assembly (2) and is connected to the inner grinding rod (4); an end of the inner grinding rod (4) away from the grinder (3) extends into the sleeve (12) and is rotatably connected to the sleeve (12) through a transmission element (5); and the transmission element (5) is connected to an external power handle through an interface (14) of the sleeve (12); and
a spiral cooling passage (16) communicated with a water injection hole (15) of the sleeve (12) is provided between an inner wall of the housing assembly (11) and an outer wall of the sleeve (12); the grinding rod assembly (2) is provided with a water inlet (21); and the water inlet (21) is communicated with the cooling passage (16) through a chamber (17) inside the housing assembly (11);
wherein the grinding rod assembly (2) comprises a fourth outer tube (22), a third outer tube (23), a second outer tube (24) and a first outer tube (25) that are arranged sequentially from inside to outside; and the fourth outer tube (22), the third outer tube (23), the second outer tube (24) and the first outer tube (25) are sequentially communicated from outside to inside to form a cooling water passage for cooling the grinder (3) and the inner grinding rod (4);
wherein a cavity is formed between each two adjacent outer tubes; the water inlet (21) is located on an outer wall of an end of the first outer tube (25) adjacent to the connecting shaft (13); a side wall of the second outer tube (24) is provided with a water guide passage (241) that is communicated with the cavity between the first outer tube (25) and the second outer tube (24); the third outer tube (23) is provided with a water guide hole (231) that is communicated with the cavity between the second outer tube (24) and the third outer tube (23); and an end of each of the fourth outer tube (22) and the third outer tube (23) adjacent to the grinder (3) is provided with a water outlet (26) that is communicated with the cavity.

2. The grinding drill with the cooling function for the minimally invasive spinal surgery according to claim 1, **characterized in that** the housing assembly (11) comprises a fastening element (111) and a water-cooled housing (112) that is detachably connected to the fastening element (111); the fourth outer tube (22), the third outer tube (23), the second outer tube (24), and the first outer tube (25) all extend into the fastening element (111) and are connected to the connecting shaft (13) through the first outer tube (25); the chamber (17) is a gap between the fastening element (111) and the connecting shaft (13); and the cooling passage (16) is located between an inner wall of the water-cooled housing (112) and the outer wall of the sleeve (12).

3. The grinding drill with the cooling function for the minimally invasive spinal surgery according to claim 2, **characterized in that** an end of the water-cooled housing (112) away from the fastening element (111) is clamped to a locking element (113) configured to lock the external power handle.

4. The grinding drill with the cooling function for the minimally invasive spinal surgery according to claim 1, **characterized in that** an outer wall of the inner grinding rod (4) is wrapped by an anti-friction tube (6); and the fourth outer tube (22) is located outside the anti-friction tube (6).

5. The grinding drill with the cooling function for the minimally invasive spinal surgery according to claim 2, **characterized in that** a gap is formed between the fourth outer tube (22) and the grinder (3).

6. The grinding drill with the cooling function for the minimally invasive spinal surgery according to any one of claims 1 to 5, **characterized in that** the grinder (3) is connected to the inner grinding rod (4) through a steel tube (31); and an end of the grinder (3) away from the inner grinding rod (4) forms an emery spherical grinding head, conical grinding head, or grinding head with a cutting edge.

7. The grinding drill with the cooling function for the minimally invasive spinal surgery according to claim 6, **characterized in that** the transmission element (5) comprises a bearing (51) provided inside the sleeve (12) and a coupling (52) in a rotational fit with the bearing (51); the coupling (52) is connected to the inner grinding rod (4); and the coupling (52) is connected to the external power handle through the interface (14).

8. The grinding drill with the cooling function for the minimally invasive spinal surgery according to claim 6, **characterized in that** a sealing ring (131) is provided between the connecting shaft (13) and the sleeve (12).

## Patentansprüche

1. Ein Schleifbohrer mit einer Kühlfunktion für eine minimalinvasive Wirbelsäulenoperation, bestehend aus einer Schleifvorrichtung (1), einer Schleifstangenanordnung (2) und einem Schleifer (3), wobei die Schleifvorrichtung (1) eine Gehäusebaugruppe (11) und eine abnehmbar in der Gehäusebaugruppe (11) befestigte Hülse (12) umfasst; ein Ende der Schleifstangenanordnung (2) in die Gehäusebaugruppe (11) hineinragt und über eine Verbindungswelle (13) mit der Hülse (12) verbunden ist; eine innere Schleifstange (4) drehbar in der Schleifstangenanordnung (2) angeordnet ist; der Schleifer (3) durch das andere Ende der Schleifstangenanordnung (2) verläuft und mit der inneren Schleifstange (4) verbunden ist; ein vom Schleifer (3) abgewandtes Ende der inneren Schleifstange (4) in die Hülse (12) hineinragt und über ein Übertragungselement (5) drehbar mit dieser verbunden ist; und das Übertragungselement (5) über eine Schnittstelle (14) der Hülse (12) mit einem externen Bedienhandgriff verbunden ist; und
zwischen einer Innenwand der Gehäusebaugruppe (11) und einer Außenwand der Hülse (12) ein spiralförmiger Kühlkanal (16) vorgesehen ist, der mit einer Wassereinspritzöffnung (15) der Hülse (12) verbunden ist; die Schleifstangenbaugruppe (2) mit einem Wassereinlass (21) versehen ist; und der Wassereinlass (21) über eine Kammer (17) innerhalb der Gehäusebaugruppe (11) mit dem Kühlkanal (16) verbunden ist;
wobei die Schleifstangenanordnung (2) ein viertes Außenrohr (22), ein drittes Außenrohr (23), ein zweites Außenrohr (24) und ein erstes Außenrohr (25) umfasst, die von innen nach außen nacheinander angeordnet sind; und das vierte Außenrohr (22), das dritte Außenrohr (23), das zweite Außenrohr (24) und das erste Außenrohr (25) von außen nach innen nacheinander miteinander verbunden sind, um einen Kühlwasserkanal zur Kühlung des Schleifers (3) und der inneren Schleifstange (4) zu bilden;
wobei zwischen je zwei benachbarten Außenrohren ein Hohlraum ausgebildet ist; der Wassereinlass (21) sich an einer Außenwand eines Endes des ersten Außenrohrs (25) in der Nähe der Verbindungswelle (13) befindet; eine Seitenwand des zweiten Außenrohrs (24) mit einem Wasserführungskanal (241) versehen ist, der mit dem Hohlraum zwischen dem ersten Außenrohr (25) und dem zweiten Außenrohr (24) in Verbindung steht; das dritte Außenrohr (23) mit einer Wasserführungsöffnung (231) versehen ist, die mit dem Hohlraum zwischen dem zweiten Außenrohr (24) und dem dritten Außenrohr (23) in Verbindung steht; und jeweils ein Ende des vierten Außenrohrs (22) und des dritten Außenrohrs (23) in der Nähe des Schleifers (3) mit einem Wasserauslass (26) versehen ist, der mit dem Hohlraum in Verbindung steht.

2. Der Schleifbohrer mit Kühlfunktion für die minimalinvasive Wirbelsäulenoperation nach Anspruch 1, **dadurch gekennzeichnet, dass** die Gehäusebaugruppe (11) ein Befestigungselement (111) und ein wassergekühltes Gehäuse (112) umfasst, das lösbar mit dem Befestigungselement (111) verbunden ist; das vierte Außenrohr (22), das dritte Außenrohr (23), das zweite Außenrohr (24) und das erste Außenrohr (25) sich alle in das Befestigungselement (111) hinein erstrecken und über das erste Außenrohr (25) mit der Verbindungswelle (13) verbunden sind, das erste Außenrohr (25); die Kammer (17) ein Spalt zwischen dem Befestigungselement (111) und der Verbindungswelle (13) ist; und der Kühlkanal (16) sich zwischen einer Innenwand des wassergekühlten Gehäuses (112) und der Außenwand der Hülse (12) befindet.

3. Der Schleifbohrer mit Kühlfunktion für die minimalinvasive Wirbelsäulenoperation nach Anspruch 2, **dadurch gekennzeichnet, dass** ein Ende des wassergekühlten Gehäuses (112), das vom Befestigungselement (111) abgewandt ist, an einem Verriegelungselement (113) befestigt ist, das zum Verriegeln des externen Kraftgriffs ausgelegt ist.

4. Der Schleifbohrer mit Kühlfunktion für die minimalinvasive Wirbelsäulenoperation nach Anspruch 1, **dadurch gekennzeichnet, dass** eine Außenwand der inneren Schleifstange (4) von einem reibungsarmen Rohr (6) umschlossen ist und das vierte Außenrohr (22) außerhalb des reibungsarmen Rohrs (6) angeordnet ist.

5. Der Schleifbohrer mit Kühlfunktion für die minimalinvasive Wirbelsäulenoperation nach Anspruch 2, **dadurch gekennzeichnet, dass** zwischen dem vierten Außenrohr (22) und dem Schleifer (3) ein Spalt ausgebildet ist.

6. Der Schleifbohrer mit Kühlfunktion für die minimalinvasive Wirbelsäulenoperation nach einem der Ansprüche 1 bis 5, **dadurch gekennzeichnet, dass** der Schleifer (3) über ein Stahlrohr (31) mit der inneren Schleifstange (4) verbunden ist und ein Ende des Schleifers (3), das von der inneren Schleifstange (4) abgewandt ist, einen kugelförmigen Schmirgelschleifkopf, einen konischen Schleifkopf oder einen Schleifkopf mit einer Schneide bildet.

7. Der Schleifbohrer mit Kühlfunktion für die minimalinvasive Wirbelsäulenoperation nach Anspruch 6, **dadurch gekennzeichnet, dass** das Übertragungselement (5) ein im Inneren der Hülse (12) angeordnetes Lager (51) und eine mit dem Lager (51) drehbar gelagerte Kupplung (52) umfasst; die Kupplung (52) mit der inneren Schleifstange (4) verbunden ist; und die Kupplung (52) über die Schnittstelle (14) mit dem externen Kraftgriff verbunden ist.

8. Der Schleifbohrer mit Kühlfunktion für die minimalinvasive Wirbelsäulenoperation nach Anspruch 6, **dadurch gekennzeichnet, dass** zwischen der Verbindungswelle (13) und der Hülse (12) ein Dichtring (131) vorgesehen ist.

## Revendications

1. Foret de meulage avec fonction de refroidissement pour une chirurgie spinale peu invasive, comprenant un accessoire de meulage (1), un ensemble tige de meulage (2) et une meuleuse (3), dans lequel l'accessoire de meulage (1) comprend un ensemble logement (11) et un manchon (12) qui est relié de manière amovible à l'intérieur de l'ensemble logement (11) ; une extrémité de l'ensemble tige de meulage (2) s'étend dans l'ensemble logement (11) et est reliée au manchon (12) via un arbre de raccordement (13) ; une tige de meulage intérieure (4) est disposée de manière rotative dans l'ensemble tige de meulage (2) ; la meuleuse (3) passe par l'autre extrémité de l'ensemble tige de meulage (2) et est reliée à la tige intérieure (4) ; une extrémité de la tige de meulage intérieure (4) éloignée de la meuleuse (3) s'étend dans le manchon (12) et est reliée de façon rotative au manchon (12) via un élément de transmission (5) ; et l'élément de transmission (5) est connecté à une poignée d'alimentation externe via une interface (14) du manchon (12) ; et
un passage de refroidissement en spirale (16) en communication avec un trou d'injection d'eau (15) du manchon (12) est prévu entre une paroi intérieure de l'ensemble logement (11) et une paroi extérieure du manchon (12) ; l'ensemble tige de meulage (2) est doté d'une entrée d'eau (21) ; et l'entrée d'eau (21) est en communication avec le passage de refroidissement (16) via une chambre (17) à l'intérieur de l'ensemble logement (11) ;
dans lequel l'ensemble tige de meulage (2) comprend un quatrième tube extérieur (22), un troisième tube extérieur (23), un deuxième tube extérieur (24) et un premier tube extérieur (25) disposés séquentiellement de l'intérieur vers l'extérieur ; et le quatrième tube extérieur (22), le troisième tube extérieur (23), le deuxième tube extérieur (24) et le premier tube extérieur (25) sont en communication séquentiellement de l'extérieur vers l'intérieur pour former un passage d'eau de refroidissement pour refroidir la meuleuse (3) et la tige de meulage intérieure (4) ;
dans lequel une cavité est formée entre les deux tubes extérieurs adjacents ; l'entrée d'eau (21) est située sur une paroi extérieure d'une extrémité du premier tube extérieur (25) adjacente à l'arbre de raccordement (13) ; une paroi latérale du deuxième tube extérieur (24) est dotée d'un passage de guidage d'eau (241) qui est en communication avec la cavité entre le premier tube extérieur (25) et le deuxième tube extérieur (24) ; le troisième tube extérieur (23) est doté d'un trou de guidage d'eau (231) en communication avec la cavité entre le deuxième tube extérieur (24) et le troisième tube extérieur (23) ; et une extrémité de chacun du quatrième tube extérieur (22) et du troisième tube extérieur (23) adjacente à la meuleuse (3) est dotée d'une sortie d'eau (26) en communication avec la cavité.

2. Foret de meulage avec la fonction de refroidissement pour la chirurgie spinale peu invasive selon la revendication 1, **caractérisé en ce que** l'ensemble logement (11) comprend un élément de fixation (111) et un logement refroidi à l'eau (112) relié de manière amovible à l'élément de fixation (111); le quatrième tube extérieur (22), le troisième tube extérieur (23), le deuxième tube extérieur (24) et le premier tube extérieur (25) s'étendent tous dans l'élément de fixation (111) et sont reliés à l'arbre de raccordement (13) via le premier tube extérieur (25) ; la chambre (17) est un espace entre l'élément de fixation (111) et l'arbre de raccordement (13) ; et le passage de refroidissement (16) se trouve entre une paroi intérieure du logement refroidi par eau (112) et la paroi extérieure du manchon (12).

3. Foret de meulage avec la fonction de refroidissement pour la chirurgie spinale peu invasive selon la revendication 2, **caractérisé en ce qu'**une extrémité du logement refroidi à l'eau (112) éloignée de l'élément de fixation (111) est fixée à un élément de verrouillage (113) configuré pour verrouiller la poignée d'alimentation externe.

4. Foret de meulage avec la fonction de refroidissement pour la chirurgie spinale peu invasive selon la revendication 1, **caractérisé en ce qu'**une paroi extérieure de la tige de meulage intérieure (4) est enroulée par un tube antifriction (6) ; et le quatrième tube extérieur (22) est situé à l'extérieur du tube antifriction (6).

5. Foret de meulage avec la fonction de refroidissement pour la chirurgie spinale peu invasive selon la revendication 2, **caractérisé en ce qu'**un espace est formé entre le quatrième tube extérieur (22) et la meuleuse (3).

6. Foret de meulage avec la fonction de refroidissement pour la chirurgie spinale peu invasive selon l'une des revendications 1 à 5, **caractérisé en ce que** la meuleuse (3) est reliée à la tige de meulage intérieure (4) par un tube en acier (31) ; et une extrémité de la meuleuse (3) éloignée de la tige de meulage intérieure (4) forme une tête de meulage sphérique, une tête conique ou une tête de meulage en émeri avec un bord de coupe.

7. Foret de meulage avec la fonction de refroidissement pour la chirurgie spinale peu invasive selon la revendication 6, **caractérisée en ce que** l'élément de transmission (5) comprend un roulement (51) disposé à l'intérieur du manchon (12) et un accouplement (52) en ajustement rotatif avec le roulement (51) ; l'accouplement (52) est relié à la tige de meulage intérieure (4) ; et l'accouplement (52) est connecté à la poignée d'alimentation externe via l'interface (14).

8. Foret de meulage avec la fonction de refroidissement pour la chirurgie spinale peu invasive selon la revendication 6, **caractérisé en ce qu'**un anneau d'étanchéité (131) est disposé entre l'arbre de raccordement (13) et le manchon (12).
